# EUROPEAN PATENT APPLICATION

(11) **EP 3 431 059 A1**
(43) Date of publication of application: **23.01.2019**
(21) Application number: 17182320.6
(22) Date of filing: 20.07.2017
(51) Int. Cl.: A61F 7/08, A61B 5/055, A61F 7/00, A61B 90/00

(54) **PATIENT SUPPORT FOR MEDICAL TREATMENT OR EXAMINATION WITH COOLING PAD**

(71) Applicant: Koninklijke Philips N.V., 5656 AE Eindhoven (NL)
(72) Inventor: KUMAR, Rakesh, 5656 AE Eindhoven (NL)
(74) Representative: de Haan, Poul Erik

(57) **Abstract**

The present invention relates to cooling the patient's body during examination

In a magnetic resonance imaging (MRI) system. According to the invention, a patient support for a magnetic resonance imaging (MRI) system (1), for supporting a patient (4) in an examination area (6) of an MRI bore (3) of the MRI system (1) is provided, the patient support (5) comprising at least one cooling pad (7) which is configured for allowing a cooling flow to go through it thereby cooling a patient's body part adjacent to the cooling pad (7). In this way, a possibility for effectively cooling a patient's body during MRI examination in a MRI bore (3) of a MRI system (1) is provided.

## Description

### FIELD OF THE INVENTION

The invention relates to the field of patient supports for supporting a patient's body, e.g. for medical examination or treatment, and especially to cooling a patient's body during examination in a magnetic resonance imaging (MRI) system, i.e. when the patient is supported by a patient support in a MRI bore of the MRI system

### BACKGROUND OF THE INVENTION

A MRI scan in a MRI bore of a MRI system usually takes approx. 30 minutes to be completed. Usually, it is difficult for the patient to stay in the same position for such a long time. Sometimes, for example if a full body scan is done, examination time may even increase to one hour. If the patient is uncomfortable during this period he may inform the MRI technician or radiologist, and then further actions may be taken by the MRI technician or the radiologist, respectively. Especially, the patient may be uncomfortable because of lying in the same position for a long time or due to rise in temperature inside the MRI bore.

It may also happen that the temperature of the patient's body or specific organs increases by 1-2 °C. In such a case the patient may feel sweat or a burning sensation on the backside of the body. In such cases it might also be helpful for the patient to pause the MRI scan for some time to give the patient some possibility to relax. In a worst case scenario, the MRI scan has to be rescheduled.

In order to address these problems US 2014/0249401 A1 describes a MRI system with a temperature control system for controlling the temperature of the patient in the examination region inside the MRI bore. This temperature control system is configured to actively control or regulate an environment of the patient and thereby the temperature or thermal comformt of the patient based upon a detected and/or an expected temperature of the patient during the MRI examination. The temperature control system includes two different cooling device for cooling the patient's body. The first cooling device is a fan or ventilator having an angular configuration for generating a forced flow of air through the MRI bore. The second cooling device comprises a cooling pad in the form of a flexible sleeve or cuff which is designed to be wrapped around the calf or calves of the patient. The pad-like sleeve or cuff is filled with a liquid that acts as a heat-transfer medium. The second cooling device further includes a supply line for supplying the heat-transfer liquid to the sleeve or cuff from a reservoir, and the return line for returning the heat-transfer liquid from the cuff to the reservoir.

### SUMMARY OF THE INVENTION

It is an object of the invention to provide an efficient possibility for cooling a patient's body during medical examination or treatment.

This object is achieved by the independent claims. Preferred embodiments are described in the sub claims.

Therefore, according to the invention, a patient support, for supporting a patient, especially for medical treatment or examination, is provided, the patient support comprising at least one cooling pad which is configured for allowing a cooling flow to go through it thereby cooling the patient's body part adjacent to the cooling pad.

Thus, in contrast to the prior art not only an airflow which is only cooling the anterior side of the patient lying on the patient support may be provided. Additionally, also the posterior side of the patient with which he is lying on the patient support may be cooled without separately fixing cooling devices to the patient. It should be noted that it may not only be the cooling effect itself which is helpful for the patient. The invention may also help in providing comfort to a patient's body with the help of the cooling flow. Of course, cooling will provide comfort to the patient when the patient is feeling heat or rise in temperature. However, since the patient may get a sensation of itching or burning because he is lying in the same position for a long period of time, providing a cooling flow will also be a solution to such an itching or burning sensation.

According to a preferred embodiment of the invention, the patient support is a patient support for a magnetic resonance imaging (MRI) system for supporting a patient in an examination area of an MRI bore of the MRI system.

In general, the cooling pad may be attached to the patient support in different manners. However, according to a preferred embodiment of the invention the cooling pad is integrated into the patient support. Therefore, according to this design the cooling pad makes an integral part of the patient support which means that the patient's comfort when supported by the patient support is not compromised by any protrusions or the like.

In this regard, according to a further preferred embodiment of the invention, the upper surface of the cooling pad is flush with the upper surface of the patient support surrounding the cooling pad. In this way, a flat surface of the patient support can be provided which means that it may also be achieved that the cooling pad is even not visible from the upper side of the patient support. In other words, the function of the patient support in order to support the patient in a MRI bore during MRI examination is not affected by the cooling pad at all.

According to the invention, it might already be advantageous if the patient support is provided with a single cooling pad. However, according to a preferred embodiment of the invention, the patient support comprises a plurality of cooling pads. These cooling pads are preferably configured and designed as the cooling pad described before. Providing the patient support with a plurality of cooling pads may provide for multiple advantages, as set out in the following.

According to a preferred embodiment of the invention, the plurality of cooling pads is arranged along the longitudinal axis of the patient support. The "longitudinal axis" of the patient support is understood to be the axis along which the patient support is introduced into a MRI bore and along which the patient is positioned on the patient support, i.e. relating to the direction from head to feet of the patient and opposite thereto. In this way, cooling pads cooling different parts of the patient's body can be controlled separately which means that the different parts of the patient's body can be cooled independently from each other. Especially, according to a preferred embodiment of the invention, the plurality of cooling pads is configured for independently cooling at least the head, the back, and the legs of the patient, respectively.

While, in general, for the cooling flow which is allowed to go through the cooling pads, a cooling liquid could be used which would not affect the image quality of a MRI scans, the design described before allows for selectively cooling only such body parts of the patient for which no MRI scan is done at the moment. Further, in between consecutive MRI scans all cooling pads may be provided with the cooling flow in order to provide for maximum cooling of the patient.

The invention also relates to a magnetic resonance imaging system with a patient support as described before. It is to be understood that such an MRI system is equipped with the usual devices used for such an MRI system like a gradient coil, a magnet, a RF transmit coil, and a RF receive coil. Further, a computer system may be provided for controlling the MRI system and for data analysis and imaging.

Further, the invention relates to a method for cooling a body part of a patient, the patient preferably being examined in a magnetic resonance imaging (MRI) system, the patient being supported on a patient support, preferably in a MRI bore of the MRI system, the method comprising the following steps:
providing a cooling pad below the patient's body part to be cooled, and
allowing a cooling flow to go through the cooling pad thereby cooling the patient's body part adjacent to the cooling pad.

This method according to the invention may also be advantageous since, according to a preferred embodiment of the invention, the following step may be comprised when used in a MRI system:
doing consecutive MRI scans, and
allowing the cooling flow to go through the cooling pad in between consecutive MRI scans.

As already set out above, in this way, no cooling fluid which would not affect the image quality of the MRI scan must be used, and due to effective cooling between MRI scans the patient may relax before the next scan begins. Therefore, according to a preferred embodiment of the invention, the cooling flow during the MRI scan is stopped.

As also set out above, according to a preferred embodiment of the invention, a plurality of cooling pads may be provided being arranged adjacent to different parts of the patient's body, the method preferably comprising the following steps:
doing a MRI scan for a specific part of the patient's body, and
allowing the cooling flow only to go through such cooling pads during the MRI scan which are different from the specific part of the patient's body the MRI scan is done for.

In this way, the cooling flow does not affect the MRI scan, either, even if no such cooling fluid is used which does not affect the image quality of the MRI scan.

The invention also relates to a non-transitory computer-readable medium, comprising instructions stored thereon, that when executed on a processor, perform the steps of:
detecting whether a magnetic resonance imaging (MRI) systems is doing a MRI scan for a specific body part of a patient being supported on a patient support in a MRI bore of the MRI system, and
allowing a cooling flow to go only through such cooling pads of a plurality of cooling pads arranged in the patient support during the MRI scan which are different from the specific part of the patient's body the MRI scan is done for.

Further, the invention relates to a non-transitory computer-readable medium, comprising instructions stored thereon, that when executed on a processor, perform the steps of:
detecting whether a magnetic resonance imaging (MRI) systems is doing a MRI scan for a patient being supported on a patient support in a MRI bore of the MRI system or not, and
allowing a cooling flow to go through at least one cooling pad arranged in the patient support when no MRI scan is done.

According to a preferred embodiment of the invention, this non-transitory computer-readable medium further comprises instructions stored thereon, that when executed on a processor, perform the additional step of:
acoustically announcing a pause time until the next MRI scan starts and/or the scan time of an upcoming MRI scan.

This predetermined time can be used for synching the cooling flow to the cooling pad and further provides the patient with certainty with respect to the upcoming situation which he would have to bear within the next minutes.

### BRIEF DESCRIPTION OF THE DRAWINGS

These and other aspects of the invention will be apparent from and elucidated with reference to the embodiments described hereinafter. Such an embodiment does not necessarily represent the full scope of the invention, however, and reference is made therefore to the claims and herein for interpreting the scope of the invention.

In the drawings:

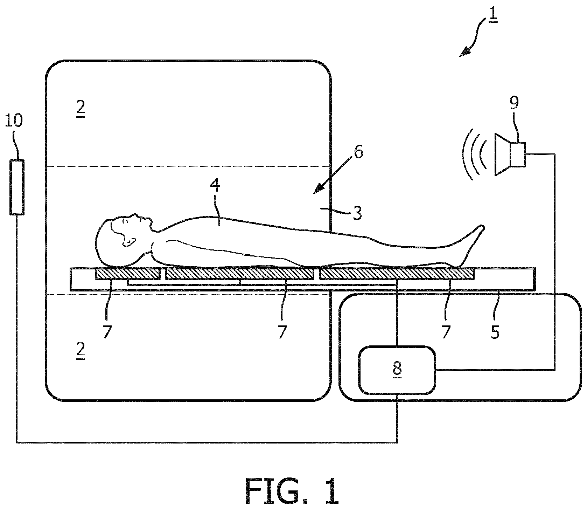

Fig. 1 schematically depicts MRI system with a patient support according to a preferred embodiment of the invention.

### DETAILED DESCRIPTION OF EMBODIMENTS

In Fig. 1 a schematic longitudinal sectional view of an MRI system 1 according to an embodiment of the invention is depicted. This MRI system 1 comprises a gradient coil, a magnet, an RF transmit coil, and an RF receive coil which are integrated into a wall area 2 surrounding a MRI bore 3 and which are not depicted in the Fig. 1 for sake of clarity. A patient 4 is positioned on a patient support 5. With this patient support 5 the patient 4 can be disposed in an examination area 6 in the MRI system 1 inside the MRI bore 3 for MRI examination.

The patient support 5 comprises three cooling pads 7 which are integrated into the patient support and which are arranged in such a way in the patient support 5 that their upper surface is flush with the upper surface of the patient support 5. The cooling pads 7 are connected to a cooling controller 8 which may provide the cooling pads 7 with a cooling flow, i.e. with a cooling fluid. Further, the cooling controller 8 controls the cooling flow to the different cooling pads 7 independently from each other. The cooling controller 8 is also connected to a speaker 9 which is used for announcing an upcoming pause time between MRI scans and the duration of an upcoming MRI scan, respectively. In this way, the patient 4 may adapt himself to the upcoming situation which helps him to relax and consciously enjoying cooling before the next MRI scan.

Further, in addition to the cooling pads 7, a fan 10 is arranged at one opening of the MRI bore 3 in order to allow for additional cooling of the patient 4. The design of the fan 10 and the cooling airstream generated thereby may follow conventional designs. An airstream generated by the fan 10 may be used additionally to the cooling by the cooling pads 7 but also in situations where cooling with the cooling pads 7 would not be appropriate and, at least temporarily, is halted.

The cooling pads 7 are arranged in such a way in the patient support 5 that they are adjacent to the head, the back, and the legs of a patient 4 lying on the patient support 5, respectively. In this way, it is possible to selectively cool only such body parts of the patient 4 which require cooling or which can be cooled without affecting the quality of the MRI scan. If image quality of the MRI scan might be affected because of the motion of cooling liquid flowing through the cooling pads 7, such cooling flow can be stopped for the respective region. For example, if the MRI scan is a head scan, then the flow to the cooling pad 7 located adjacent to the head of the patient 4 can be stopped by the cooling controller 8.

Further, the temperature of the cooling flow can also be controlled by the cooling controller 8. Thus, it is not only possible to switch the cooling flow on or off, but the present preferred embodiment of the invention depicted in Fig. 1 also allows for fine tuning of the cooling temperature in order to achieve a comfortable situation for the patient 4.

If a cooling fluid would affect the image quality of the MRI scan only airflow could be used.

A cooling flow provided in between consecutive MRI scans would help to relax the patient and help him in long scan session like full body scans.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**REFERENCE SYMBOL LIST**

| | |
|---|---|
| 1 | MRI system |
| 2 | wall area |
| 3 | MRI bore |
| 4 | patient |
| 5 | patient support |
| 6 | examination area |
| 7 | cooling pads |
| 8 | cooling controller |
| 9 | speaker |

## Claims

1. A patient support, for supporting a patient (4), the patient support (5) comprising at least one cooling pad (7) which is configured for allowing a cooling flow to go through it thereby cooling a patient's body part adjacent to the cooling pad (7).

2. The patient support according to claim 1, wherein the cooling pad (7) is integrated into the patient support (5).

3. The patient support according to claim 2, wherein the upper surface of the cooling pad (7) is flush with the upper surface of the patient support (5) surrounding the cooling pad.

4. The patient support according to any of the preceding claims, wherein the patient support (5) comprises a plurality of cooling pads (7).

5. The patient support according to claim 4, wherein the plurality of cooling pads (7) are arranged along the longitudinal axis of the patient support (5).

6. The patient support according to claim 5, wherein the plurality of cooling pads (7) are configured for cooling at least the head, back, and legs or the patient (4), respectively.

7. A magnetic resonance imaging system with a patient support (5) according to any of the preceding claims.

8. Method for cooling a body part of a patient (4), the patient being supported on a patient support (5) in an MRI bore (3) of the MRI system (1), the method comprising the following steps:
providing a cooling pad (7) below the patient's body part to be cooled, and
allowing a cooling flow to go through the cooling pad (5) thereby cooling the patient's body part adjacent to the cooling pad (7).

9. Method according to claim 8, with the following step:
examining the patient (4) in a magnetic resonance imaging (MRI) system (1),
supporting the patient (4) on the patient support (5) in an MRI bore (3) of the MRI system (1),
doing consecutive MRI scans, and
allowing the cooling flow to go through the cooling pad (7) in between consecutive MRI scans.

10. Method according to claim 9, with the following step:
stopping the cooling flow during a MRI scan.

11. Method according to any claim 9 or 10, with the following steps:
providing a plurality of cooling pads (7) being arranged adjacent to different parts of the patient's body,
doing a MRI scan for a specific part of the patient's body, and
allowing the cooling flow only to go through such cooling pads (7) during the MRI scan which are different from the specific part of the patient's body the MRI scan is done for.

12. A non-transitory computer-readable medium, comprising instructions stored thereon, that when executed on a processor, perform the steps of:
detecting whether a magnetic resonance imaging (MRI) systems (1) is doing a MRI scan for a specific body part of a patient (4) being supported on a patient support (5) in a MRI bore (3) of the MRI system (1), and
allowing a cooling flow to go only through such cooling pads (7) of a plurality of cooling pads (7) arranged in the patient support (5) during the MRI scan which are different from the specific part of the patient's body the MRI scan is done for.

13. A non-transitory computer-readable medium, comprising instructions stored thereon, that when executed on a processor, perform the steps of:
detecting whether a magnetic resonance imaging (MRI) system (1) is doing a MRI scan for a patient (4) being supported on a patient support (5) in a MRI bore (3) of the MRI system (1) or not, and
allowing a cooling flow to go through at least one cooling pad (7) arranged in the patient support (5) when no MRI scan is done.

14. The non-transitory computer-readable medium according to claim 13, comprising instructions stored thereon, that when executed on a processor, perform the step of:
at the end of an MRI scan, acoustically announcing a pause time until the next MRI scan starts, and/or
before a MRI scan, acoustically announcing a scan time of the next MRI scan.
